# EUROPEAN PATENT APPLICATION

(11) **EP 0 819 393 A1**
(43) Date of publication of application: **21.01.1998**
(21) Application number: 97112217.1
(22) Date of filing: 17.07.1997
(51) Int. Cl.: A45D 40/00, A61F 13/00, A61F 13/15

(54) **Makeup remover pad**

(30) Priority: 19.07.1996 IT MI960520 U
(71) Applicant: Industrie Cartarie Tronchetti S.p.A., 55023 Borgo a Mozzano (Lucca) (IT)
(72) Inventor: Tronchetti, Sauro, 55100 Lucca (IT)
(74) Representative: Forattini, Amelia

(57) **Abstract**

Makeup remover pad having a central body (7) made of absorbent material, preferably carded cotton, which lies between two supporting layers (3,5) made of non-woven fabric.

## Description

The present invention relates to a makeup remover pad.

Cotton pads for removing makeup and generally suitable to be used for cleansing have long been commercially available.

Cotton pads are used both as wipe and/or applicators, for cosmetic products, such as oils or solutions, and also in the medical field.

These makeup remover pads must have essentially two characteristics: they must be absorbent and they must not flake or produce lint.

Currently commercially available pads are generally made of carded cotton and not all of them fully meet the expected requirements.

US-5,230,119 discloses a disposable laminated pad constituted by a base pad, made of nonwoven fiber, associated with an impervious shield having an handle. The purpose of the shield is to prevent contact between the user and the fluids to be applied or removed.

JP-A-3-286726 discloses a cosmetic wiping cloth constituted by a nonwoven polyolefin sheet which provides good oil absorbing speed and affinity in general. This type of pad is not ideal for aqueous solutions.

EP-A-0441667 discloses a makeup removing pad made of a waterproof layer between two layers of absorbent material of the same thickness. The purpose of the design of this pad is to allow the use of different fluids on the same pad. The absorbing properties of this type of pad are not ideal.

An aim of the present invention is to provide a makeup remover pad having functional characteristics which are optimum and identical to, or better than, those of commercially available pads.

A further object of the invention is to provide a makeup remover pad which can be produced with conventional equipment and with competitive production costs.

This aim, these objects and others which will become apparent hereinafter are achieved by a makeup remover pad, as defined in the appended claims.

Further characteristics and advantages will become apparent from the description of the makeup remover pad, illustrated only by way of non-limitative example in the accompanying drawings, wherein:
FIG. 1 is a perspective view of the pad, shown opened out;
FIG. 2 is a sectional side view of the pad;
FIG. 3 is a perspective view of the pad.

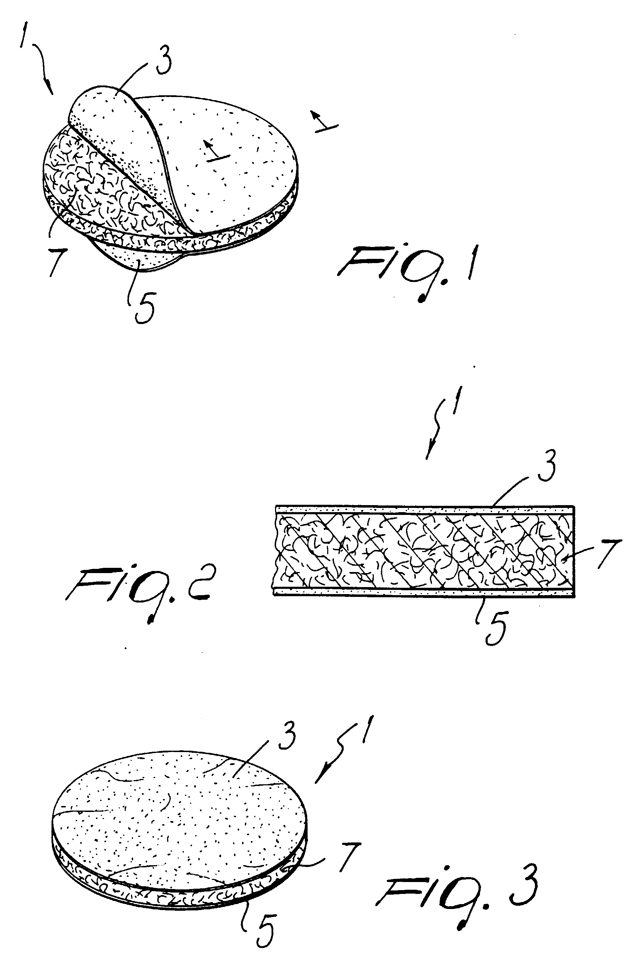

With reference to the above figures, the makeup remover pad according to the invention, generally designated by the reference numeral 1, includes a central body 7 made of absorbent material which lies between at least two supporting layers 3 and 5.

The central body 7 is preferably made of carded cotton, while the layers 3 and 5 are constituted by nonwoven fabric, preferably cotton.

The layers 3 and 5 are preferably fixed to the central body by embossing, associating two rolls of nonwoven fabric in the immediate vicinity of the carder which is suitable to produce the central body. This system optimizes waste and is more efficient.

The layers 3 and 5 can also be glued to the central body.

Preferred sizes of the pad are a 6 cm diameter and a 2.5 mm thickness.

In practice, it has been observed that the invention achieves the intended aim and objects, a makeup remover pad having been provided which is more resistant and does not form lint.

The makeup remover pad according to the invention does not tear easily, remains unchanged during use and also has a considerable absorbent power.

Compared to conventional makeup remover pads, the pad according to the present invention has a considerably improved stress resistance, both when dry and soaked (from 2 to 10 times greater than conventional pads, according to stress tests).

The nonwoven cotton fabric has substantially different properties compared to natural cotton, namely the nonwoven cotton fabric has a greater consistency when dry and especially when wet.

The pad according to the invention is therefore particularly suited to be used in wet conditions and provides a greater quantity of detergent because its absorption is retarded.

However, since a good makeup remover must also absorb the fluids to be removed, the inner layer, or central body, provides for a through absorption when pressure is applied on the pad and the fluids are allowed to cross the outer layers.

## Claims

1. Makeup remover pad, characterized in that it comprises a central body (7) made of absorbent material associated with two supporting layers of nonwoven fabric.

2. Disk according to claim 1, characterized in that said central body is made of cotton.

3. Disk according to claim 1, characterized in that said nonwoven fabric is made of cotton.

4. Disk according to claim 1, characterized in that said central body is made of carded cotton.

5. Disk according to one or more of the preceding claims, characterized in that said supporting layers are associated with said central body by embossing.

6. Disk according to one or more of the preceding claims, characterized in that said supporting layers are glued to said central body.
